(19) Europäisches Patentamt
European Patent Office
Office européen des brevets

(11) Veröffentlichungsnummer: **0 374 559 A2**

# (12) EUROPÄISCHE PATENTANMELDUNG

(21) Anmeldenummer: **89122287.9**

(22) Anmeldetag: **02.12.89**

(51) Int. Cl.5: **C07D 333/38, C07C 329/06, A01N 47/06**

(30) Priorität: **17.12.88 DE 3842626**

(43) Veröffentlichungstag der Anmeldung: **27.06.90 Patentblatt 90/26**

(84) Benannte Vertragsstaaten: **BE CH DE ES FR GB GR IT LI NL**

(71) Anmelder: **BAYER AG**
**D-5090 Leverkusen 1 Bayerwerk(DE)**

(72) Erfinder: **Daum, Werner, Dr.**
**Baerenstrasse 18**
**D-4150 Krefeld 1(DE)**
Erfinder: **Bielefeldt, Dietmar, Dr.**
**Beuthener Strasse 13**
**D-4030 Ratingen(DE)**
Erfinder: **Brandes, Wilhelm, Dr.**
**Eichendorffstrasse 3**
**D-5653 Leichlingen(DE)**

## (54) Substituierte 2-Phenyl-2,2-difluorethylthiolcarbonate, Verfahren zu ihrer Herstellung und ihre Verwendung zur Bekämpfung von Schädlingen.

(57) Substituierte 2-Phenyl-2,2-difluorethylthiolcarbonate der Formel (I)

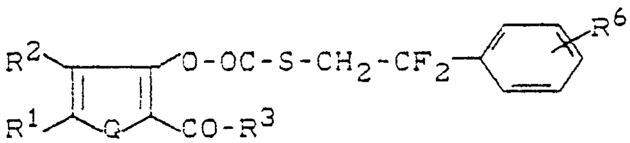

(I)

in welcher
R', $R^2$, $R^3$ und $R^5$ die in der Beschreibung angegebenen Bedeutungen haben und ihre Verwendung zur Bekämpfung von Schädlingen.
Die neuen Verbindungen sind durch die Formel (I) allgemein beschrieben und sie können nach bekannten Verfahren hergestellt werden, z.B. indem man geeignete aromatische oder heteroaromatische Hydroxylverbindungen mit geeigneten Acylierungsmitteln oder geeignete Chlorcarbonyloxyaromaten oder Chlorcarbonyloxyheteroaromaten mit geeigneten Mercaptanen umsetzt.

EP 0 374 559 A2

## Substituierte 2-Phenyl-2,2-difluorethylthiolcarbonate, Verfahren zu ihrer Herstellung und ihre Verwendung zur Bekämpfung von Schädlingen

Die vorliegende Erfindung betrifft neue substituierte 2-Phenyl-2,2-difluorethylthiolcarbonate, Verfahren zu ihrer Herstellung und ihre Verwendung zur Bekämpfung von Schädlingen, insbesondere von Pilzen.

Es sind bereits 2-Phenyl-2,2-difluorethylthiolcarbamate mit herbiziden Eigenschaften bekannt (vgl. DE-OS 3 341 515 und DE-OS 3 341 516). Weiterhin ist bekannt, daß 2,5-Bis-(alkoxycarbonyl)-3,4-bis-(acyloxy)-thiophene und 2,5-Bis-(alkoxycarbonyl)-3-alkyl-4-acyloxythiophene fungizide Eigenschaften besitzen (vgl. EP 32 748 und EP 93 384).

Weiterhin ist das 2,5-Bis-(isopropoxycarbonyl)-3-methyl-4-(3-methylbenzoyloxy)-thiophen zu nennen, das bekannt ist aus T. Wada et al., Proceedings af the 10th Intern. Congress of Plant Protection, 20.-25.11.1983, Brighton, Vol 1, 400-407.

Weiterhin sind Acylthiophen-Derivate mit fungiziden Eigenschaften bekannt, wie z.B. 2,5-Bis-(isopropoxycarbonyl)-4-methyl-3-(3-trifluormethylthiobenzoyloxy)-thiophen (vgl. DE-OS 3 402 625).

Es wurden neue substituierte 2-Phenyl-2,2-difluorethylthiolcarbonate der allgemeinen Formel (I)

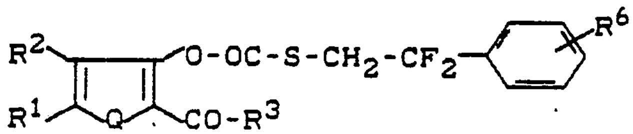

(I)

in welcher

$R^1$ für Wasserstoff, Alkoxycarbonyl, Alkoxyalkoxycarbonyl, Alkylthioalkoxycarbonyl, Halogenalkoxycarbonyl, Cyanalkoxycarbonyl, Alkenoxycarbonyl, Alkinoxycarbonyl, Cycloalkyloxycarbonyl oder für Cycloalkylalkoxycarbonyl steht,

$R^2$ für Wasserstoff, Alkyl oder für Phenyl steht oder

$R^1$ und $R^2$ gemeinsam für den Butadien-1,4-diyl-Rest stehen,

$R^3$ für Alkoxy, Alkoxyalkoxy, Alkylthioalkoxy, Fluoralkoxy, Cyanalkoxy, Alkenoxy, Alkinoxy, Cycloalkyloxy, Cycloalkylalkoxy oder für die Gruppe

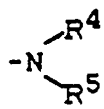

steht, in der

$R^4$ für Alkyl, Alkoxyalkyl, Alkylthioalkyl, Cyanalkyl, Fluoralkyl, Alkenyl, Alkinyl oder Cycloalkyl steht,

$R^5$ für Wasserstoff, Alkyl, Alkoxyalkyl, Alkylthioalkyl, Fluoralkyl, Cyanalkyl, Alkenyl, Alkinyl oder Cycloalkyl steht,

oder

$R^4$ und $R^5$ gemeinsam mit dem Stickstoffatom, an dem sie stehen, für einen unsubstituierten oder substituierten Heterocyclus stehen, welcher in der Alkylenkette durch weitere Heteroatome substituiert sein kann,

$R^6$ für Wasserstoff, Methyl oder Chlor steht und

Q für Schwefel oder -CH=CH- steht,

gefunden.

Weiterhin wurde gefunden, daß man die neuen 2-Phenyl-2,2-difluorethylthiolcarbonate der allgemeinen Formel (I)

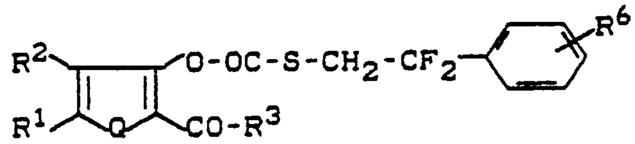

(I)

in welcher

$R^1$ für Wasserstoff, Alkoxycarbonyl, Alkoxyalkoxycarbonyl, Alkylthioalkoxycarbonyl, Halogenalkoxycarbonyl, Cyanalkoxycarbonyl, Alkenoxycarbonyl, Alkinoxycarbonyl, Cycloalkyloxycarbonyl oder Cycloalkylalkoxycarbonyl steht,

$R^2$ für Wasserstoff, Alkyl oder für Phenyl steht oder

$R^1$ und $R^2$ gemeinsam für den Butadien-1,4-diyl-Rest stehen,

$R^3$ für Alkoxy, Alkoxyalkoxy, Alkylthioalkoxy, Fluoralkoxy, Cyanalkoxy, Alkenoxy, Alkinoxy, Cycloalkyloxy, Cycloalkylalkoxy oder für die Gruppe

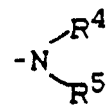

steht, in der

$R^4$ für Alkyl, Alkoxyalkyl, Alkylthioalkyl, Cyanalkyl, Fluoralkyl, Alkenyl, Alkinyl oder Cycloalkyl steht,

$R^5$ für Wasserstoff, Alkyl, Alkoxyalkyl, Alkylthioalkyl, Fluoralkyl, Cyanalkyl, Alkenyl, Alkinyl oder Cycloalkyl steht,

oder

$R^4$ und $R^5$ gemeinsam mit dem Stickstoffatom, an dem sie stehen, für einen unsubstituierten oder substituierten Heterocyclus stehen, welcher in der Alkylenkette durch weitere Heteroatome substituiert sein kann,

$R^6$ für Wasserstoff, Methyl oder Chlor steht und

Q für Schwefel oder -CH=CH- steht,

erhält, wenn man

a) eine aromatische oder heteroaromatische Hydroxylverbindung der allgemeinen Formel (II)

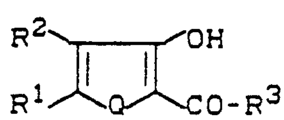

(II)

in welcher

$R^1$, $R^2$, $R^3$ und Q die oben angegebene Bedeutung haben,

oder deren Ammonium-, Alkali- oder Erdalkalisalze, mit einem Acylierungsmittel der Formel (III)

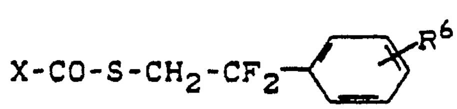

(III)

in welcher

$R^6$ die angegebene Bedeutung hat und

X für eine Abgangsgruppe, wie z.B. Halogen steht,

umsetzt

oder indem man

b) Verbindungen der Formel (II) erst mit Phosgen in Gegenwart einer tertiären organischen Base umsetzt und dann den gebildeten Chlorcarbonyloxy-aromaten bzw. Chlorcarbonyloxy-heteroaromaten der Formel (IV)

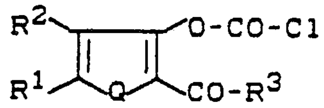

(IV)

in welcher
$R^1$, $R^2$, $R^3$ und Q die oben angegebenen Bedeutungen haben,
mit einem 2-Phenyl-2,2-difluorethylmercaptan der Formel (V)

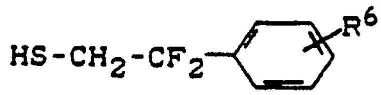

(V)

in welcher
$R^6$ die obengenannte Bedeutung hat,
in Gegenwart einer organischen oder anorganischen Base und in Gegenwart von Lösungs- und Verdünnungsmitteln umsetzt
oder indem man
c) für den Fall, daß in der Formel (I)
$R^2$ für Alkyl oder Phenyl steht,
Q für Schwefel steht,
$R^6$ für Wasserstoff, Methyl oder Chlor steht und
$R^1$ und $-COR^3$ Estergruppen mit ungleichen Resten sind,
ein 4-Hydroxythiophenderivat der Formel (IIa)

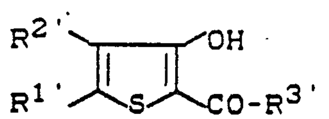

(IIa).

in welcher
$R^{1'}$ für Alkoxycarbonyl, Alkoxyalkoxycarbonyl, Alkylthioalkoxycarbonyl, Halogenalkoxy carbonyl, Cyanalkoxycarbonyl, Alkenoxycarbonyl, Alkinoxycarbonyl, Cycloalkyloxycarbonyl oder Cycloalkylalkoxycarbonyl steht,
$R^{2'}$ für Alkyl oder Phenyl steht und
$R^{3'}$ für Alkoxy, Alkoxyalkoxy, Alkylthioalkoxy, Fluoralkoxy, Cyanalkoxy, Alkenoxy, Alkinoxy, Cycloalkyloxy oder Cycloalkylalkoxy steht,
mit der Maßgabe, daß $R^{1'}$ und $COR^{3'}$ für gleiche Estergruppen stehen, mit wäßrig alkoholischer Alkalilauge im Molverhältnis 1:2 partiell zu 2-Carboxy-4-hydroxy-5-carbonsäureestern der Formel (IIb)

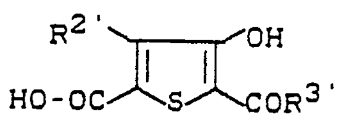

(IIb)

in welcher,
$R^{2'}$ und $R^{3'}$ die bei der Formel (IIa) angegebenen Bedeutungen haben,
verseift und die Verbindungen der Formel (IIb) nach ihrer Isolierung mit einem 2-Phenyl-2,2-difluorethylthiolcarbonylhalogenid der Formel (III) in Gegenwart einer tertiären organischen Base zu Verbindungen der Formel (VI)

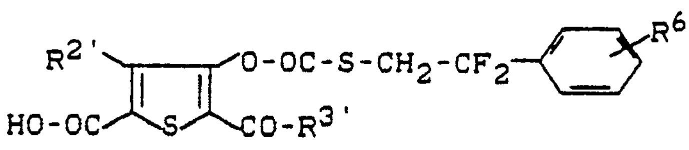

(VI)

in welcher
$R^{2'}$, $R^{3'}$ und $R^6$ die oben angegebenen Bedeutungen haben,
umsetzt und die so erhaltenen Verbindungen der Formel (VI) zu Säurechloriden der Formel (VII)

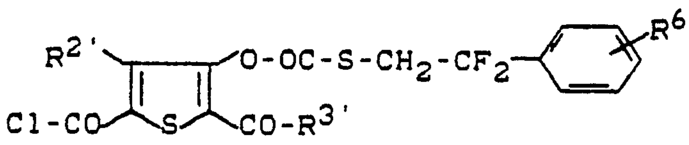

(VII)

in welcher
$R^{2'}$, $R^{3'}$ und $R^6$ die angegebenen Bedeutungen haben,
umsetzt und diese Verbindungen in letzter Stufe mit einem Alkohol der Formel (VIII)
$R^1$ OH (VIII)
in welcher
$R^1$ für Alkyl, Alkoxyalkyl, Alkylthioalkyl, Halogenalkyl, Cyanalkyl, Alkenyl, Alkinyl, Cycloalkyl oder Cycloalkylalkyl steht,
in Gegenwart einer organischen Base verestert.

Weiterhin wurde gefunden, daß die neuen substituierten 2-Phenyl-2,2-difluorethylthiolcarbonate der Formel (I) starke Wirkungen gegen Schädlinge aufweisen, besonders gegen Pilze.

Überraschenderweise zeigen die erfindungsgemäßen substituierten 2-Phenyl-2,2-difluorethylthiolcarbonate der Formel (I) eine bessere Wirksamkeit gegen phytopathogene Pilze bei ausgezeichneter Pflanzenverträglichkeit als die aus dem Stand der Technik bekannten konstitutionell ähnlichen Verbindungen gleicher Wirkungsrichtung.

Die erfindungsgemäßen substituierten 2-Phenyl-2,2-difluorethylthiolcarbonate sind durch die Formel (I) allgemein definiert. Bevorzugt sind Verbindungen der Formel (I), in welcher
$R^1$ für Wasserstoff, für Alkoxycarbonyl mit 1-7 Kohlenstoffatomen, für Alkoxyalkoxycarbonyl oder für Alkylthioalkoxycarbonyl mit je 1-5 Kohlenstoffatomen je Alkylteil, für Halogenalkoxycarbonyl mit 1-5 Kohlenstoffatomen und 1-5 gleichen oder verschiedenen Halogenatomen, für Cyanalkoxycarbonyl mit 1-5 Kohlenstoffatomen im Alkylteil, für Alkenoxycarbonyl mit 3 oder 4 Kohlenstoffatomen im Alkenteil, für Alkinoxycarbonyl mit 3-5 Kohlenstoffatomen im Alkinteil, für Cycloalkyloxycarbonyl mit 4-6 Kohlenstoffatomen im Cycloalkylteil oder Cycloalkylalkoxycarbonyl mit 4-8 Kohlenstoffatomen im Cycloalkylalkoxyteil steht,
$R^2$ für Wasserstoff, für Alkyl mit 1-4 Kohlenstoffatomen oder für Phenyl steht oder
$R^1$ und $R^2$ zusammen für den Butadien-1,4-diyl-Rest stehen,
$R^3$ für Alkoxy mit 1-6 Kohlenstoffatomen, für Alkoxyalkoxy oder für Alkylthioalkoxy mit je 1-5 Kohlenstoffatomen je Alkylteil, für Fluoralkoxy mit 1-5 Kohlenstoffatomen und 1-5 Fluoratomen, für Cyanalkoxy mit 1-5 Kohlenstoffatomen im Alkylteil, für Alkenoxy mit 3 oder 4 Kohlenstoffatomen, für Alkinoxy mit 3-5 Kohlenstoffatomen, für Cycloalkyloxy mit 4-6 Kohlenstoffatomen oder für Cycloalkylalkoxy mit 4-8 Kohlenstoffatomen oder für die Gruppe

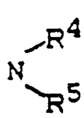

steht, in der
$R^4$ für Alkyl mit 1-5 Kohlenstoffatomen, für Alkoxyalkyl oder Alkylthioalkyl mit jeweils 1-5 Kohlenstoffatomen je Alkylteil, für Cyanalkyl mit 1-5 Kohlenstoffatomen im Alkylteil, für Fluoralkyl mit 1-3 Fluoratomen und 1-5 Kohlenstoffatomen, für Alkenyl oder Alkinyl mit je 3-5 Kohlenstoffatomen oder Cycloalkyl mit 3-6 Kohlenstoffatomen steht,
$R^5$ für Wasserstoff, Alkyl mit 1-5 Kohlenstoffatomen, für Alkoxyalkyl oder Alkylthioalkyl mit jeweils 1-5

Kohlenstoffatomen je Alkylteil, für Cyanalkyl mit 1-5 Kohlenstoffatomen im Alkylteil, für Fluoralkyl mit 1-3 Fluoratomen und 1-5 Kohlenstoffatomen, für Alkenyl oder Alkinyl mit 3-5 Kohlenstoffatomen oder Cyclohexyl steht, oder

$R^4$ und $R^5$ zusammen mit dem Stickstoffatom, an dem sie stehen, für einen 5-, 6- oder 7-gliedrigen heterocyclischen Ring stehen, der weitere ein bis drei, gleiche oder verschiedene Heteroatome - wie Aza-, Oxa- oder Thiaelemente - enthalten und durch Alkylgruppen mit 1-4 Kohlenstoffatomen gleich oder verschieden, ein- bis dreifach substituiert sein kann,

$R^6$ für Wasserstoff, Methyl oder Chlor steht und

Q für Schwefel oder für -CH = CH- steht.

Besonders bevorzugt sind Verbindungen der Formel (I), in welcher

$R^1$ für Wasserstoff, Methoxycarbonyl, Ethoxycarbonyl, n- oder Isopropoxycarbonyl, 2,2-Dimethylpropoxycarbonyl, 2-Methoxyethoxycarbonyl, 2-Ethoxyethoxycarbonyl, 2-Methylthioethoxycarbonyl, 2-Ethylthioethoxycarbonyl, 2,2,2-Trifluorethoxycarbonyl, Cyanmethoxycarbonyl, Cyanethoxycarbonyl, Allyloxycarbonyl, Methallyloxycarbonyl, 2-Propinyloxycarbonyl, 1,1-Dimethyl-2-propinyloxycarbonyl, Cyclopentyloxycarbonyl, Cyclohexyloxycarbonyl oder Cyclohexylmethoxycarbonyl steht,

$R^2$ für Wasserstoff, Methyl, Ethyl, n-Propyl, Isopropyl, n-Butyl, sek.-Butyl, iso-Butyl, tert.-Butyl oder Phenyl steht oder

$R^1$ und $R^2$ gemeinsam für den Butadien-1,4-diyl-Rest stehen,

$R^3$ für Methoxy-, Ethoxy-, n- oder Isopropoxy-, sec.-Butoxy-, 2,2-Dimethylpropoxy-, 2-Methoxyethoxy-, 2-Ethoxyethoxy-, 2-Methylthioethoxy-, 2-Ethylthioethoxy-, 2,2,2-Trifluorethoxy-, Cyanmethoxy-, Cyanethoxy-, Allyloxy-, Methallyloxy-, 2-Propinyloxy-, 1,1-Dimethyl-2-propinyloxy-, Cyclopentyloxy-, Cyclohexyloxy-, Cyclohexylmethoxy- oder für die Gruppe

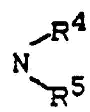

steht, in welcher

$R^4$ für Methyl, Ethyl, n- oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyanethyl, ω-Cyanpentyl, Allyl, Methallyl, 2-Propinyl, 1,1-Dimethyl-2-propinyl, Cyclopropyl, Cyclobutyl, Cyclopentyl oder Cyclohexyl steht,

$R^5$ für Wasserstoff, Methyl, Ethyl, n- oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyanoethyl, ω-Cyanpentyl, Allyl, Methallyl, 2-Propinyl, 1,1-Dimethyl-2-propinyl oder Cyclohexyl steht, oder

$R^4$ und $R^5$ zusammen mit dem Stickstoffatom, an dem sie stehen, für Pyrrolidin, Piperidin, 2-Methylpiperidin, 3-Methylpiperidin, 4-Methylpiperidin, Hexahydro-1H-azepin, Morpholin, 2,6-Dimethylmorpholin, Thiazolidin, N'-Methylpiperazin oder N'-Propylpiperazin stehen,

$R^6$ für Wasserstoff, Methyl oder Chlor steht und

Q für Schwefel oder für -CH = CH- steht.

Ganz besonders bevorzugt seien Verbindungen der Formel (I) genannt, in welcher

$R^1$ für Wasserstoff, Ethoxycarbonyl, Isopropoxycarbonyl, sek.-Butoxycarbonyl oder Cyclopentyloxycarbonyl steht,

$R^2$ für Wasserstoff, Methyl, Isopropyl oder Phenyl steht,

$R^3$ für Ethoxy, Isopropoxy, sek.-Butoxy, Cyclopentyloxy oder für die Gruppe

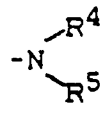

steht, in welcher

$R^4$ für Methyl oder Methoxyethyl steht und

$R^5$ für Wasserstoff steht oder

$R^4$ und $R^5$ zusammen für Pentan-1,5-diyl oder für 3-Oxa-pentan-1,5-diyl stehen,

$R^6$ für Wasserstoff, Methyl oder Chlor steht und

Q für Schwefel oder für -CH = CH- steht.

Weiterhin sind Verbindungen der Formel (I) genannt, in welcher
$R^1$ und $R^2$ gemeinsam für den Butadien-1,4-diyl-Rest stehen,
$R^3$ für Ethoxy, Isopropoxy, sek.-Butoxy, Cyclopentyloxy oder für die Gruppe

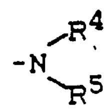

steht, in welcher
$R^4$ für Methyl oder Methoxyethyl steht,
$R^5$ für Wasserstoff oder Methyl steht oder
$R^4$ und $R^5$ zusammen für Pentan-1,5-diyl oder für 3-Oxa-pentan-1,5-diyl stehen,
$R^6$ für Wasserstoff, Methyl oder Chlor steht und
Q für Schwefel oder für -CH=CH- steht.

Außerdem sind insbesondere Verbindungen der Formel (I) genannt, in welcher
$R^1$ für Wasserstoff, Ethoxycarbonyl, Isopropoxycarbonyl, sek.-Butoxycarbonyl oder Cyclopentyloxycarbonyl steht,
$R^2$ für Wasserstoff, Methyl, Isopropyl oder Phenyl steht,
$R^3$ für Ethoxy, Isopropoxy, sek.-Butoxy oder Cyclopentyloxy steht,
$R^6$ für Wasserstoff oder Methyl steht und
Q für -CH=CH- steht.

Weitere insbesondere Verbindungen der Formel (I) seien genannt, in welcher
$R^1$ für Wasserstoff, Ethoxycarbonyl, Isopropoxycarbonyl, sek.-Butoxycarbonyl oder Cyclopentyloxycarbonyl steht,
$R^2$ für Wasserstoff, Methyl, Isopropyl oder Phenyl steht,
$R^3$ für Ethoxy, Isopropoxy, sek.-Butoxy oder Cyclopentyloxy steht,
$R^6$ für Wasserstoff, Methyl steht und
Q für Schwefel steht.

Für die Substituenten $R^{1'}$, $R^{1''}$, $R^{2'}$ und $R^{3'}$ gelten für die bevorzugten, besonders bevorzugten und insbesondere bevorzugten Definitionen die jeweiligen Teile von $R^1$, $R^2$ und $R^3$.

Die Alkylreste als solche oder in Zusammensetzungen, wie Alkoxy, Alkylthioalkoxy, Cyanalkyl u.a., können jeweils geradkettig oder verzweigt sein.

Sind die aromatischen und/oder heteroaromatischen Reste substituiert, verstehen wir darunter eine ein- bis fünffache, vorzugsweise ein- bis dreifache, gleiche oder verschiedene Substitution, falls nicht anders angegeben.

Verwendet man als Ausgangsstoffe zur Herstellung der erfindungsgemäßen Verbindungen der Formel (I) nach Verfahrensvariante a) 2,5-Bis-(cyclopentyloxycarbonyl)-3-phenyl-4-hydroxythiophen und 2,2-Difluor-2-(4-chlorphenyl)-ethylthiolcarbonylfluorid und Pyridin als Lösungsmittel und als Fluorwasserstoffakzeptor, so kann der Reaktionsverlauf durch das folgende Formelschema wiedergegeben werden:

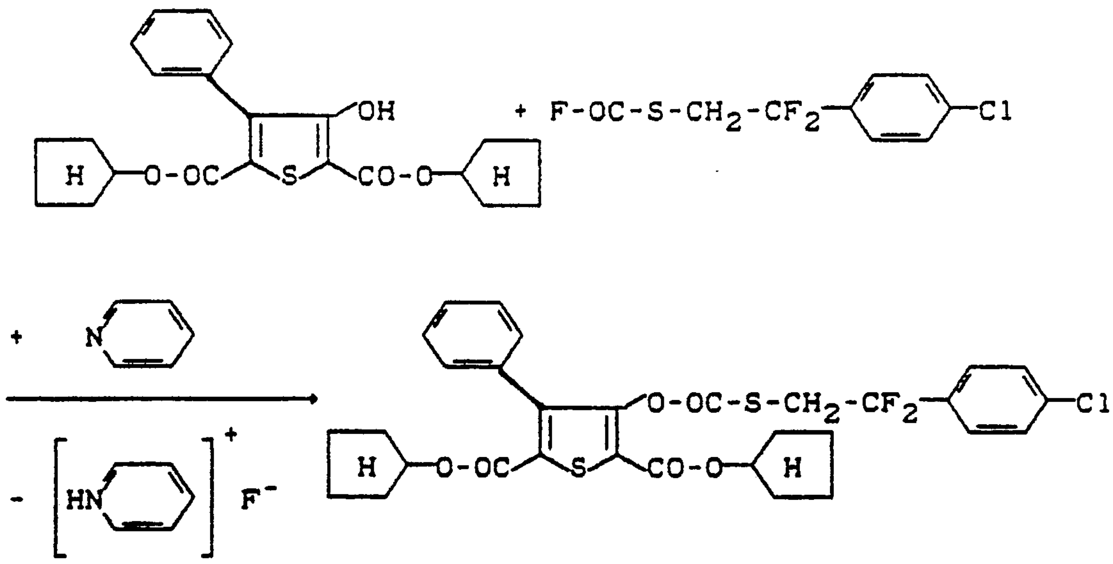

Verwendet man als Ausgangsstoffe zur Herstellung der erfindungsgemäßen Verbindungen der Formel (I) nach Verfahrensvariante b) z.B. 2,5-Bis-(cyclopentyloxycarbonyl)-3-phenyl-4-hydroxythiophen und Phosgen in Gegenwart einer tertiären organischen Base in einem 1. Schritt und in einem 2. Schritt 2-Phenyl-2,2-difluorethylmercaptan, so kann der Reaktionsverlauf durch das folgende Formelschema wiedergegeben werden:

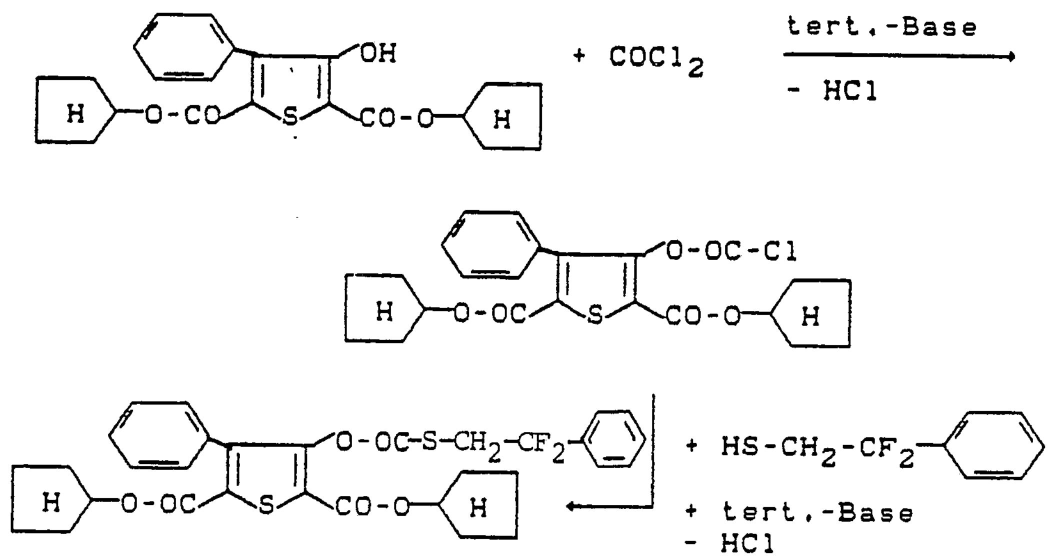


Verwendet man als Ausgangsstoffe zur Herstellung erfindungsgemäßer Verbindungen der Formel (I) nach Verfahrensvariante c) z.B. 2,5-Bis-(i-propyloxycarbonyl)-3-phenyl-4-hydroxythiophen und wäßrige alkoholische Alkalilauge in einem 1. Schritt, setzt dann mit 2-Phenyl-2,2-difluorethylthiolcarbonylchlorid um in einem 2. Schritt und anschließend mit z.B. Oxalylchlorid in Dimethylformamid in Gegenwart einer organischen Base und anschließend mit sek.-Butanol in Gegenwart einer organischen Base, so kann der Reaktionsverlauf durch das folgende Formelschema wiedergegeben werden:

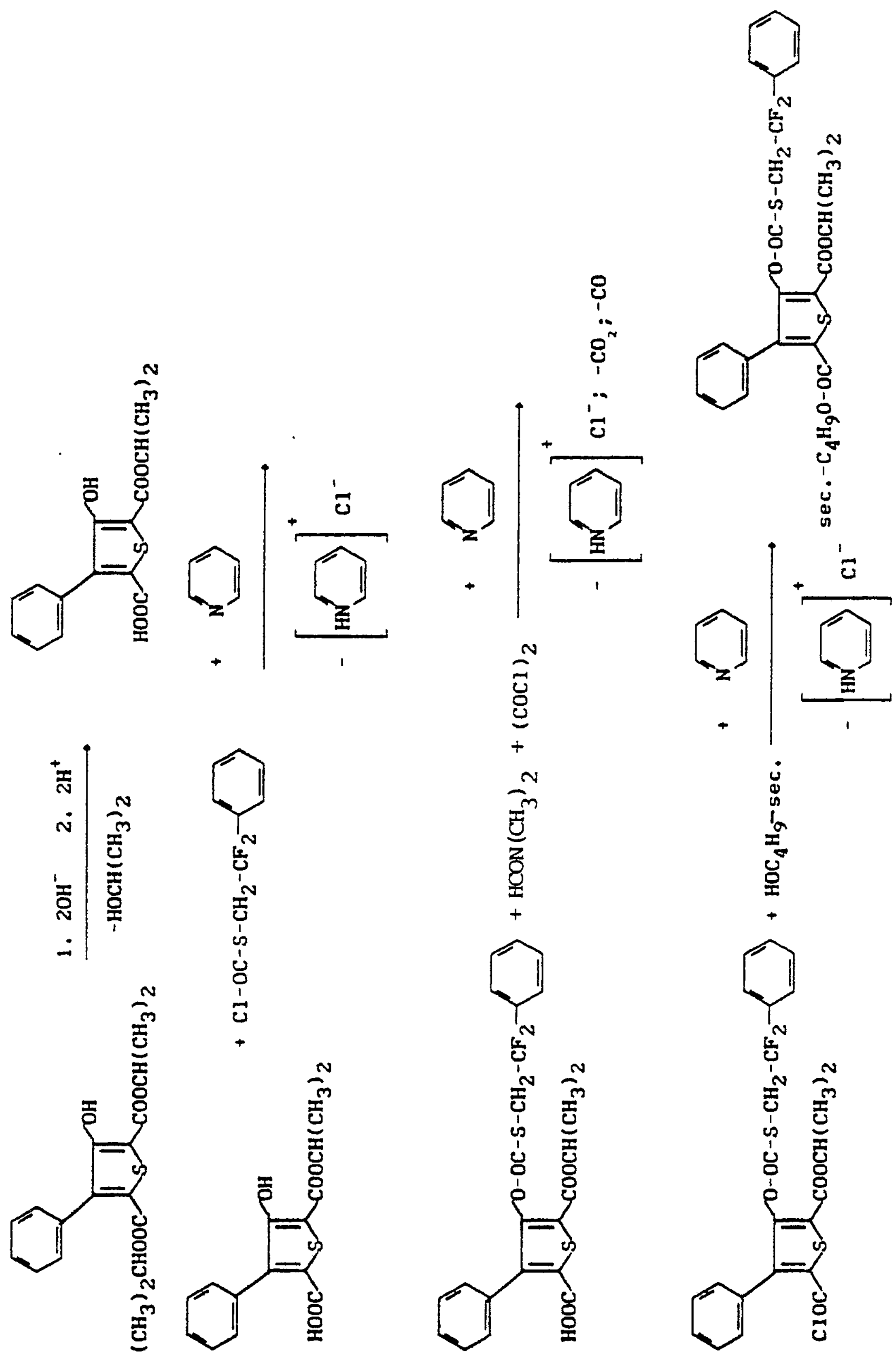


Die als Ausgangsstoffe für die erfindungsgemäßen Verfahrensvarianten a), b) und c) benötigten aromatischen oder heteroaromatischen Hydroxylverbindungen sind durch die Formeln (II) bzw. (IIa) bzw. (IIb) allgemein definiert. Die Verbindungen sind überwiegend bekannt und können nach bekannten Verfahren hergestellt werden. Zu nennen sind hier insbesondere:

Salicylsäure-isopropyl-, -sek.-butyl- und -cyclopentyl-ester und Salicylsäure-pyrrolidid;

1-Hydroxynaphthoesäure-2-ethyl-, -isopropyl-, -n-propyl- und -cyclopentyl-ester und 1-Hydroxynaphthoesäure-2-morpholid, -piperidid und -pyrrolidid; 3-Hydroxythiophen-2-carbonsäure-methyl-, -ethyl-, -isopropyl- und -sek.-butyl-ester;
2,5-Bis-(methoxycarbonyl)-, 2,5-Bis-(ethoxycarbonyl)-, 2,5-Bis-(isopropoxycarbonyl)-, 2,5-Bis-(butyloxycarbonyl)- und 2,5-Bis-(sek.-butyloxycarbonyl)-3-hydroxythiophen;
2,5-Bis-(methoxycarbonyl)-, 2,5-Bis-(ethoxycarbonyl)-, 2,5-Bis-(isopropoxycarbonyl)-, 2,5-Bis-(propoxycarbonyl)-, 2,5-Bis-(sek.-butoxycarbonyl)-, 2,5-Bis-(isobutoxycarbonyl)-, 2,5-Bis-(pentoxycarbonyl)-, 2,5-Bis-(cyclopentyloxycarbonyl)- und 2,5-Bis-(cyclohexylmethoxycarbonyl)-3-methyl-, -3-isopropyl- und -3-phenyl-4-hydroxythiophen;
2-Methoxycarbonyl-3-methyl-4-hydroxythiophen-5-carbonsäure-N-methylamid;
2-Ethoxycarbonyl-3-methyl-4-hydroxythiophen-5-carbonsäure-N-methylamid, -N,N-dimethylamid, -N-(2-methoxyethyl)-amid, -N-(3-methoxypropyl)-amid, -pyrrolidid, -piperidin und -morpholid;
2-Isopropoxycarbonyl-3-methyl-4-hydroxythiophen-5-carbonsäure-N-methylamid, -N,N-dimethylamid, -N-(2-methoxyethyl)-amid, -N-(3-methoxypropyl)-amid, -N-(2-ethoxyethyl)-amid,-N-tert.-butylamid, -pyrrolidid, -piperidid und -morpholid;
2-Propoxycarbonyl-3-methyl-4-hydroxythiophen-5-carbonsäuremorpholid und -piperidid;
2-Butoxycarbonyl-3-methyl-4-hydroxy-thiophen-5-carbonsäure-N-(2-methoxyethyl)-amid und -N-(3-methoxypropyl)-amid;
2-sek.-Butoxycarbonyl-3-methyl-4-hydroxythiophen-5-carbonsäure-N-(2-methoxyethyl)-amid, -N-(2-ethoxyethyl)-amid, -N-(3-methoxypropyl)-amid, -morpholid, -piperidid und -pyrrolidid;
2-Isobutoxycarbonyl-3-methyl-4-hydroxythiophen-5-carbonsäure-morpholid und -piperidid;
2-Cyclopentyloxycarbonyl-3-methyl-4-hydroxythiophen-5-carbonsäure-N-(2-methoxyethyl)-amid, -morpholid und -piperidid;
2-Ethoxycarbonyl-3-isopropyl-4-hydroxythiophen-5-carbonsäuremorpholid, -piperidid und -pyrrolidid;
2-Isopropoxycarbonyl-3-isopropyl-4-hydroxythiophen-5-carbonsäure-N-(2-methoxyethyl)-amid, -morpholid, -piperidid und -pyrrolidid;
2-n-Propoxycarbonyl-3-isopropyl-4-hydroxythiophen-5-carbonsäure-N-(2-methoxyethyl)-amid, -morpholid und -piperidid;
2-Methoxycarbonyl-3-phenyl-4-hydroxythiophen-5-carbonsäure-morpholid und -piperidid;
2-Ethoxy-, 2-Isopropoxy-, 2-Propoxy-, 2-sek.-Butoxy-, 2-Isobutoxy- und 2-Cyclopentyloxy-carbonyl-3-phenyl-4-hydroxythiophen-5-carbonsäure-N-methylamid, -N,N-dimethylamid, -N-(2-methoxyethyl)-amid, -N-(2-ethoxyethyl)-amid, -N-(3-methoxypropyl)-amid, -morpholid, -piperidid und -pyrrolidid.

Hydroxythiophencarbonsäurederivate sind z.B. bekannt aus DE-OS 3 523 313 und 3 602 889, Ber. 89, 1897 (1936), Indian J. of Chem. 11, 313-314 (1973), EP-PS 93 384.

Das für Verfahrensvariante b) benötigte Phosgen ist ein großtechnisch herstellbarer, bekannter Stoff, ebenso wie die bei Verfahrensvariante c) genannten Alkohole der Formel (VIII).

Weiterhin werden für die Umsetzungen zu den erfindungsgemäßen Verbindungen der Formel (I) 2-Phenyl-2,2-difluorethylthiolcarbonylhalogenide bei den Verfahrensvarianten a) und c) benötigt, die durch die Formel (III) allgemein definiert sind. Die Herstellung dieser Verbindungen ist aus DE-OS 3 341 515 und 3 341 516 bekannt.

Als Verdünnungsmittel für die erfindungsgemäße Umsetzung eines aromatischen oder heteroaromatischen Hydroxycarbonsäurederivats der Formeln (II) bzw. (IIa) mit einem Acylierungsmittel der Formel (III) nach Verfahrensvarianten a) und c) kommen alle gegenüber den Reaktionspartnern inerten organischen Lösungsmittel in Frage; vorzugsweise werden polare Lösungsmittel verwendet. Zu nennen sind hier beispielsweise Acetonitril, Aceton, Chloroform, Benzonitril, Dimethylacetamid, Dimethylformamid, Dimethylsulfoxid, Chlorbenzol, Essigsäureethylester, Dioxan, Methylethylketon, Methylenchlorid und Tetrahydrofuran.

Als Säurebinder für die Umsetzung werden organische Basen verwendet, vorzugsweise tertiäre Amine. Zu nennen sind hier: Chinolin, Dimethylbenzylamin, Dimethylanilin, Ethyldicyclohexylamin, Ethyldiisopropylamin, Picolin, Pyridin und Triethylamin.

Man kann auch einen Überschuß an tertiärem Amin zugleich als Lösungsmittel verwenden. Auch kann man zur Durchführung des erfindungsgemäßen Verfahrens Alkali- oder Erdalkalisalze der umzusetzenden aromatischen oder heteroatomatischen Hydroxycarbonsäurederivate in einem inerten Lösungsmittel vorlegen, oder man erzeugt das Salz, indem man in eine Mischung aus dem aromatischen oder heteroaromatischen Hydroxycarbonsäurederivat und einem hochsiedenden Lösungsmittel Alkalilauge, Alkoholate oder eine entsprechende Erdalkaliverbindung gibt und dann vorsichtig entwässert, bzw. den Alkohol abdestilliert oder man gibt ein Alkali- oder Erdalkalihydrid hinzu. Auch kann es zweckmäßig sein, die Reaktion in Gegenwart eines Katalysators wie, z.B. 4-Dimethylaminopyridin durchzuführen.

Die Reaktionen können auch in heterogenen Systemen bestehend aus Wasser und einem mit Wasser nicht mischbaren Lösungsmittel durchgeführt werden.

Die Reaktionstemperaturen und die Reaktionsdauer werden durch die Aktivität der Ausgangsprodukte bestimmt. Im allgemeinen arbeitet man etwa zwischen -50 und +80°C, vorzugsweise zwischen -10 und +60°C.

Zur Umsetzung eines aromatischen oder heteroaromatischen Hydroxycarbonsäurederivats der Formel (II) erst mit Phosgen und anschließend mit einem Mercaptan der Formel (V) nach Verfahren b) wird in einer ersten Stufe eine Mischung bestehend aus einem aromatischen oder heteroaromatischen Hydroxycarbonsäurederivat der Formel (II), einem tertiären Amin, wie z.B. Chinolin, Dimethylbenzylamin, Dimethylanilin, Ethyldicyclohexylamin, Ethyldiisopropylamin, Picolin, Pyridin oder Triethylamin, und einem inerten organischen Lösungsmittel, wie z.B. Methylenchlorid, Chloroform, Chlorbenzol, Ethylacetat, Toluol oder Xylol, zu überschüssigem Phosgen, gelöst in dem inerten organischen Lösungsmittel, gegeben. Dabei arbeitet man im allgemeinen zwischen -50 und +80°C, vorzugsweise bei 0 bis +30°C.

Nach Isolierung des 4-Chlorcarbonyloxy-Derivats der Formel (IV) wird dieses mit einem Mercaptan der Formel (V) in Gegenwart einer organischen oder anorganischen Base oder mit einem Salz des Mercaptans der Formel (V) umgesetzt.

Für diese Umsetzung kommen im Prinzip mit Wasser mischbare oder nicht mischbare Lösungsmittel, wie vorstehend aufgeführt, in Frage. Im allgemeinen arbeitet man bei Temperaturen entsprechend der Phosgenierungsreaktion.

Die Abgangsgruppe X in der Formel (III) ist vorzugsweise Halogen. Die Verbindungen der Formeln (IV), (V), (VI) und (VII) sind entweder bekannt oder analog nach bekannten Verfahren herstellbar.

Bei der Verfahrensvariante c) werden zur Herstellung der Carbonylchloride Säurechloride, wie z.B. Oxalylchlorid, verwendet.

Je nach Arbeitsbedingungen fallen die erfindungsgemäßen Wirkstoffe kristallin aus oder sie bleibem im organischen Lösungsmittel gelöst und können dann nach Auswaschen der Lösung mit Wasser durch vorsichtiges Einengen der Lösung oder durch Zugabe wenig polarer organischer Lösungsmittel, wie Cyclohexan, Dibutylether, Diisopropylether oder Tetrachlorkohlenstoff, abgeschieden werden. Gegebenenfalls müssen mit Wasser mischbare polare Lösungsmittel nach der Reaktion durch Abdampfen im Vakuum entfernt werden.

Sind die erfindungsgemäßen Verbindungen in einem mit Wasser mischbaren Lösungsmittel gelöst, so können sie auch durch Zugabe von Wasser ausgefällt werden.

Die erfindungsgemäßen Verbindungen zersetzen sich zum Teil bei höherer Temperatur; in diesen Fällen können die Schmelzpunkte nur mit geringer Genauigkeit oder überhaupt nicht ermittelt werden. Das Vorliegen bestimmter Strukturelemente ist aus den NMR-Spektren ersichtlich.

Die erfindungsgemäßen Wirkstoffe weisen eine starke biologische Wirkung auf und können zur Bekämpfung von Schädlingen praktisch eingesetzt werden. Die Wirkstoffe sind somit u.a. für den Gebrauch als Pflanzenschutzmittel, insbesondere als Fungizide, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;

Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalo cyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0.1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%,

vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die erfindungsgemäßen Verbindungen der Formel (I) besitzen eine besonders gute Wirkung gegen pilzliche Schädlinge im Obst- und Gemüseanbau, erwähnt sei ferner die Wirkung gegen Schädlinge im Reisanbau.

Herstellungsbeispiele

Beispiel 1

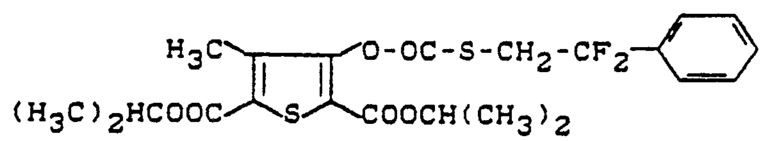

14 g 2,5-Bis-(isopropoxycarbonyl)-3-methyl-4-hydroxythiophen (0,049 Mol) und 0,02 g 4-Dimethylaminopyridin werden in 100 ml trockenem Pyridin bei 20 °C vorgelegt. Man tropft 12 g 2-Phenyl-2,2-difluorethylthiolcarbonylfluorid (0,054 Mol) hinzu und hält die Reaktionsmischung dann 4 Stunden auf 60 °C. Es werden 250 ml Ethylacetat hinzugegeben, man wäscht einmal mit eiskaltem Wasser, einmal mit verdünnter Sodalösung, einmal mit Wasser und trocknet über Natriumsulfat. Die Reaktionslösung wird im Wasserstrahlvakuum eingedampft. Der Abdampfrückstand wird an Kieselgel 60 mit Methylcyclohexan/Toluol/Essigsäure im Volumenverhältnis 3/5/1 chromatographiert. Die Produktfraktion wird eingedampft und der Abdampfrückstand bei 60 °C/0,1 mbar getrocknet. Man erhält 17 g 2,5-Bis-(isopropoxycarbonyl)-3-methyl-4-(2-phenyl-2,2-difluorethylthiocarbonyloxy)-thiophen als viskose Masse.

$^1$H-NMR ($CDCl_3$): COOCH 5,20 (m, 2H); $CH_3$ 2,26 (s, 3H); S-$CH_2$-$CF_2$ 3,71 (t, 2H)

Die Verbindung kristallisiert aus Petrolether Fp. 66,5 °C.

Auf gleiche Weise wie in Beispiel 1 bzw. in der Beschreibung angegeben werden die Verbindungen der Formel (I) gemäß den Beispielen 2 bis 9 erhalten:

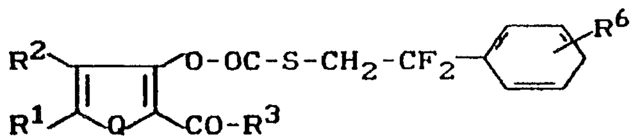

(I)

| Beispiel Nr. | $R^1$ | $R^2$ | $R^3$ | $R^6$ | Q | Physikalische Daten |
|---|---|---|---|---|---|---|
| 2 | $-COOC_2H_5$ | $CH_3$ | 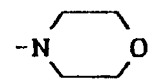 | H | S | Fp.: 68° C |
| 3 | $-COOC_3H_7$-n | $C_6H_5$ | 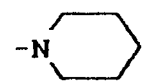 | H | S | *) $^1$H-NMR ($CDCl_3$): $COOCH_2$ 4,07 (t,2H); 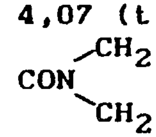 + $SCH_2CF_2$ 3,35-3,65 (m;6H) |
| 4 | $-COOC_2H_5$ | $C_6H_5$ | $-OC_2H_5$ | H | S | Fp.: 121° C |
| 5 | H | H | $-OCH(CH_3)_2$ | H | -CH=CH- | *) $^1$H-NMR ($CDCl_3$): COOCH 5,22 (m,1H); $SCH_2CF_2$ 3,69 (t,2H) |

| Beispiel Nr. | $R^1$ | $R^2$ | $R^3$ | $R^6$ | Q | Physikalische Daten |
|---|---|---|---|---|---|---|
| 6 | $-COOCH(CH_3)_2$ | $CH(CH_3)_2$ | $-OCH(CH_3)_2$ | H | S | *) $^1H$-NMR ($CDCl_3$): COOCH 5,18 (m,2H); $SCH_2CF_2$ 3,7 (m,2H) |
| 7 | $-COOCH(CH_3)_2$ | $CH_3$ | 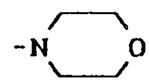 | H | S | *) $^1H$-NMR ($CDCl_3$): COOCH 5,20 (m, 1H); $N-CH_2-CH_2-O$ + $SCH_2CF_2$ 3,4-3,8 (m,10H) |
| 8 | $-COOCH(CH_3)_2$ | $C_6H_5$ | $-OCH(CH_3)_2$ | H | S | *) $^1H$-NMR ($CDCl_3$): COOCH 5,06 + 5,22 (2m,2H); $SCH_2CF_2$ 3,58 (t, 2H) |
| 9 | $-COOCH(CH_3)_2$ | $CH_3$ | 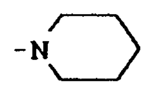 | H | S | *) $^1H$-NMR ($CDCl_3$): COOCH 5,19 (m,1H); $SCH_2CF_2$ 3,66 (t,2H) |

| Beispiel Nr. | $R^1$ | $R^2$ | $R^3$ | $R^6$ | Q | Physikalische Daten |
|---|---|---|---|---|---|---|
| 10 | H | H | $-OCH(CH_3)_2$ | H | S | *) $^1$H-NMR ($CDCl_3$): COOCH 5,18 (m,1H); $SCH_2CF_2$ 3,70 (t,2H) |
| 11 | $-COOC_2H_5$- | $CH_3$ | $-OC_2H_5$ | H | S | *) $^1$H-NMR ($CDCl_3$): $COOCH_2$ 4,34 (m, 4H); $SCH_2CF_2$ 3,70 (t, 2H) |
| 12 | $-COOCH(CH_3)C_2H_5$ | $CH_3$ | $-OCH(CH_3)C_2H_5$ | H | S | *) $^1$H-NMR ($CDCl_3$): COOCH 5,04 (m, 2H); $SCH_2CF_2$ 3,71 (t, 2H) |
| 13 | -COO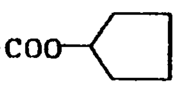 | $CH_3$ | -O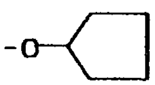 | H | S | Fp.: 95,5° C |
| 14 | $-COOCH(CH_3)_2$ | $CH_3$ | $-OCH(CH_3)_2$ | $CH_3$ | S | *) $^1$H-NMR ($CDCl_3$): COOCH 5,21 (m,2H); $SCH_2CF_2$ 3,69 (t,2H); ar-$CH_3$ 2,39 (s,3H); hetar-$CH_3$ 2,28 (s,3H) |

*viskoses Produkt

Anwendungsbeispiele

In den folgenden Anwendungsbeispielen werden die nachfolgend aufgeführten Verbindungen als Vergleichssubstanzen eingesetzt:

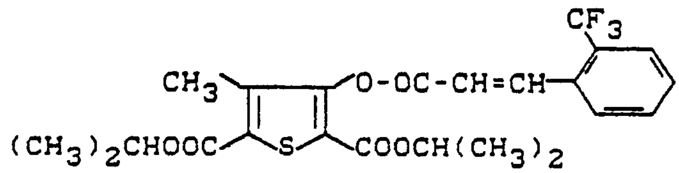

(A)

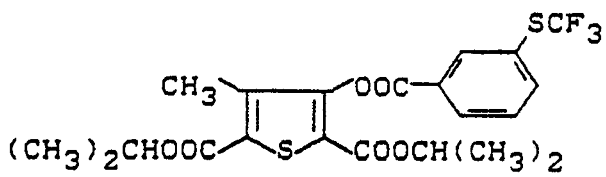

(B)

bekannt aus DE 3 402 625.

Beispiel A

Podosphaera-Test (Apfel) / protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Podosphaera leucotricha bestäubt.

Die Pflanzen werden anschließend bei 23° C und bei einer relativen Luftfeuchtigkeit von ca. 70% im Gewächshaus aufgestellt.

9 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen 1 und 3.

Beispiel B

Sphaerotheca-Test (Gurke) protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen 1 und 3.

## Ansprüche

1. Substituierte 2-Phenyl-2,2-difluorethylthiolcarbonate der allgemeinen Formel (I)

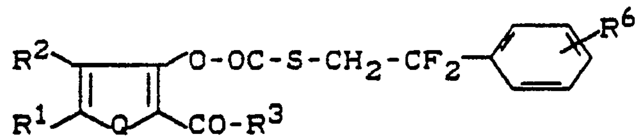

(I)

in welcher

$R^1$ für Wasserstoff, Alkoxycarbonyl, Alkoxyalkoxycarbonyl, Alkylthioalkoxycarbonyl, Halogenalkoxycarbonyl, Cyanalkoxycarbonyl, Alkenoxycarbonyl, Alkinoxycarbonyl, Cycloalkyloxycarbonyl oder für Cycloalkylalkoxycarbonyl steht,

$R^2$ für Wasserstoff, Alkyl oder für Phenyl steht oder

$R^1$ und $R^2$ gemeinsam für den Butadien-1,4-diyl-Rest stehen,

$R^3$ für Alkoxy, Alkoxyalkoxy, Alkylthioalkoxy, Fluoralkoxy, Cyanalkoxy, Alkenoxy, Alkinoxy, Cycloalkyloxy, Cycloalkylalkoxy oder für die Gruppe

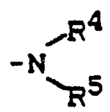

steht, in der

$R^4$ für Alkyl, Alkoxyalkyl, Alkylthioalkyl, Cyanalkyl, Fluoralkyl, Alkenyl, Alkinyl oder Cycloalkyl steht,

$R^5$ für Wasserstoff, Alkyl, Alkoxyalkyl, Alkylthioalkyl, Fluoralkyl, Cyanalkyl, Alkenyl, Alkinyl oder Cycloalkyl steht,

oder

$R^4$ und $R^5$ gemeinsam mit dem Stickstoffatom, an dem sie stehen, für einen unsubstituierten oder substituierten Heterocyclus stehen, welcher in der Alkylenkette durch weitere Heteroatome substituiert sein kann,

$R^6$ für Wasserstoff , Methyl oder Chlor steht und

Q für Schwefel oder -CH=CH- steht.

2. Substituierte 2-Phenyl-2,2-difluor-ethylthiolcarbonate gemäß Anspruch 1, wobei in Formel (I)

$R^1$ für Wasserstoff, für Alkoxycarbonyl mit 1-7 Kohlenstoffatomen, für Alkoxyalkoxycarbonyl oder für Alkylthioalkoxycarbonyl mit je 1-5 Kohlenstoffatomen je Alkylteil, für Halogenalkoxycarbonyl mit 1-5 Kohlenstoffatomen und 1-5 gleichen oder verschiedenen Halogenatomen, für Cyanalkoxycarbonyl mit 1-5 Kohlenstoffatomen im Alkylteil, für Alkenoxycarbonyl mit 3 oder 4 Kohlenstoffatomen im Alkenteil, für Alkinoxycarbonyl mit 3-5 Kohlenstoffatomen im Alkinteil, für Cycloalkyloxycarbonyl mit 4-6 Kohlenstoffatomen im Cycloalkylteil oder Cycloalkylalkoxycarbonyl mit 4-8 Kohlenstoffatomen im Cycloalkylalkoxyteil steht,

$R^2$ für Wasserstoff, für Alkyl mit 1-4 Kohlenstoffatomen oder für Phenyl steht oder

$R^1$ und $R^2$ zusammen für den Butadien-1,4-diyl-Rest stehen,

$R^3$ für Alkoxy mit 1-6 Kohlenstoffatomen, für Alkoxyalkoxy oder für Alkylthioalkoxy mit je 1-5 Kohlenstoffatomen je Alkylteil, für Fluoralkoxy mit 1-5 Kohlenstoffatomen und 1-5 Fluoratomen, für Cyanalkoxy mit 1-5 Kohlenstoffatomen im Alkylteil, für Alkenoxy mit 3 oder 4 Kohlenstoffatomen, für Alkinoxy mit 3-5 Kohlenstoffatomen, für Cycloalkyloxy mit 4-6 Kohlenstoffatomen oder für Cycloalkylalkoxy mit 4-8 Kohlenstoffatomen oder für die Gruppe

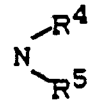

steht, in der
$R^4$ für Alkyl mit 1-5 Kohlenstoffatomen, für Alkoxyalkyl oder Alkylthioalkyl mit jeweils 1-5 Kohlenstoffatomen je Alkylteil, für Cyanalkyl mit 1-5 Kohlenstoffatomen im Alkylteil, für Fluoralkyl mit 1-3 Fluoratomen und 1-5 Kohlenstoffatomen, für Alkenyl oder Alkinyl mit je 3-5 Kohlenstoffatomen oder Cycloalkyl mit 3-6 Kohlenstoffatomen steht,
$R^5$ für Wasserstoff, Alkyl mit 1-5 Kohlenstoffatomen, für Alkoxyalkyl oder Alkylthioalkyl mit jeweils 1-5 Kohlenstoffatomen je Alkylteil, für Cyanalkyl mit 1-5 Kohlenstoffatomen im Alkylteil, für Fluoralkyl mit 1-3 Fluoratomen und 1-5 Kohlenstoffatomen, für Alkenyl oder Alkinyl mit 3-5 Kohlenstoffatomen oder Cyclohexyl steht, oder
$R^4$ und $R^5$ zusammen mit dem Stickstoffatom, an dem sie stehen, für einen 5-, 6- oder 7-gliedrigen heterocyclischen Ring stehen, der weitere ein bis drei, gleiche oder verschiedene Aza-, Oxa-oder Thiaelemente enthalten und durch Alkylgruppen mit 1-4 Kohlenstoffatomen gleich oder verschieden, ein- bis dreifach substituiert sein kann,
$R^6$ für Wasserstoff, Methyl oder Chlor steht und
Q für Schwefel oder für -CH=CH- steht.

3. Substituierte 2-Phenyl-2,2-difluorethylthiolcarbonate gemäß Anspruch 1, wobei in der Formel (I)
$R^1$ für Wasserstoff , Methoxycarbonyl, Ethoxycarbonyl, n- oder Isopropoxycarbonyl, 2,2-Dimethylpropoxycarbonyl, 2-Methoxyethoxycarbonyl, 2-Ethoxyethoxycarbonyl, 2-Methylthioethoxycarbonyl, 2-Ethylthioethoxycarbonyl, 2,2,2-Trifluorethoxycarbonyl, Cyanmethoxycarbonyl, Cyanethoxycarbonyl, Allyloxycarbonyl, Methallyloxycarbonyl, 2-Propinyloxycarbonyl, 1,1-Dimethyl-2-propinyloxycarbonyl, Cyclopentyloxycarbonyl, Cyclohexyloxycarbonyl oder Cyclohexylmethoxycarbonyl steht,
$R^2$ für Wasserstoff, Methyl, Ethyl, n-Propyl, Isopropyl, n-Butyl, sek.-Butyl, iso-Butyl, tert.-Butyl oder Phenyl steht oder
$R^1$ und $R^2$ gemeinsam für den Butadien-1,4-diyl-Rest stehen,
$R^3$ für Methoxy-, Ethoxy-, n- oder Isopropoxy-, sec.-Butoxy-, 2,2-Dimethylpropoxy-, 2-Methoxyethoxy-, 2-Ethoxyethoxy-, 2-Methylthioethoxy-, 2-Ethylthioethoxy-, 2,2,2-Trifluorethoxy-, Cyanmethoxy-, Cyanethoxy-, Allyloxy-, Methallyloxy-, 2-Propinyloxy-, 1,1-Dimethyl-2-propinyloxy-, Cyclopentyloxy-, Cyclohexyloxy-, Cyclohexylmethoxy- oder für die Gruppe

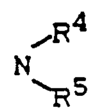

steht, in welcher
$R^4$ für Methyl, Ethyl, n- oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyanethyl, ω-Cyanpentyl, Allyl, Methallyl, 2-Propinyl, 1,1-Dimethyl-2-propinyl, Cyclopropyl, Cyclobutyl, Cyclopentyl oder Cyclohexyl steht,
$R^5$ für Wasserstoff, Methyl, Ethyl, n-oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyanoethyl, ω-Cyanpentyl, Allyl, Methallyl, 2-Propinyl, 1,1-Dimethyl-2-propinyl oder Cyclohexyl steht, oder
$R^4$ und $R^5$ zusammen mit dem Stickstoffatom, an dem sie stehen, für
Pyrrolidin, Piperidin, 2-Methylpiperidin, 3-Methylpiperidin, 4-Methylpiperidin, Hexahydro-1H-azepin, Morpholin, 2,6-Dimethylmorpholin, Thiazolidin, N'-Methylpiperazin oder N'-Propylpiperazin stehen,
$R^6$ für Wasserstoff, Methyl oder Chlor steht und
Q für Schwefel oder für -CH=CH-steht.

4. Substituierte 2-Phenyl-2,2-difluorethylthiolcarbonate gemäß Anspruch 1, wobei in der Formel (I)
$R^1$ für Wasserstoff, Ethoxycarbonyl, Isopropoxycarbonyl, sek.-Butoxycarbonyl oder Cyclopentyloxycarbonyl steht,
$R^2$ für Wasserstoff, Methyl, Isopropyl oder Phenyl steht,
$R^3$ für Ethoxy, Isopropoxy, sek.-Butoxy, Cyclopentyloxy oder für die Gruppe

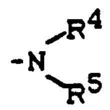

steht, in welcher
$R^4$ für Methyl oder Methoxyethyl steht und
$R^5$ für Wasserstoff oder Methyl steht oder
$R^4$ und $R^5$ zusammen für Pentan-1,5-diyl oder für 3-Oxa-pentan-1,5-diyl stehen,
$R^6$ für Wasserstoff, Methyl oder Chlor steht und
Q für Schwefel oder für -CH=CH- steht.

5. Substituierte 2-Phenyl-2,2-difluorethylthiolcarbonate gemäß Anspruch 1, wobei in der Formel (I)
$R^1$ und $R^2$ gemeinsam für den Butadien-1,4-diyl-Rest stehen,
$R^3$ für Ethoxy, Isopropoxy, sek.-Butoxy, Cyclopentyloxy oder für die Gruppe

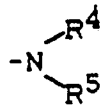

steht, in welcher
$R^4$ für Methyl oder Methoxyethyl steht,
$R^5$ für Wasserstoff oder Methyl steht oder
$R^4$ und $R^5$ zusammen für Pentan-1,5-diyl oder für 3-Oxa-pentan-1,5-diyl stehen,
$R^6$ für Wasserstoff, Methyl oder Chlor steht und
Q für Schwefel oder für -CH=CH- steht.

6. Verfahren zur Herstellung von substituierten 2-Phenyl-2,2-difluor-ethylthiolcarbonaten der allgemeinen Formel (I)

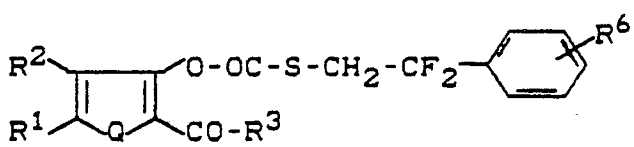

in welcher
R' für Wasserstoff, Alkoxycarbonyl, Alkoxyalkoxycarbonyl, Alkylthioalkoxycarbonyl, Halogenalkoxycarbonyl, Cyanalkoxycarbonyl, Alkenoxycarbonyl, Alkinoxycarbonyl, Cycloalkyloxycarbonyl oder Cycloalkylalkoxycarbonyl steht,
$R^2$ für Wasserstoff, Alkyl oder für Phenyl steht oder
R' und $R^2$ gemeinsam für den Butadien-1,4-diyl-Rest stehen,
$R^3$ für Alkoxy, Alkoxyalkoxy, Alkylthioalkoxy, Fluoralkoxy, Cyanalkoxy, Alkenoxy, Alkinoxy, Cycloalkyloxy, Cycloalkylalkoxy oder für die Gruppe

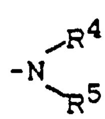

steht, in der
$R^4$ für Alkyl, Alkoxyalkyl, Alkylthioalkyl, Cyanalkyl, Fluoralkyl, Alkenyl, Alkinyl oder Cycloalkyl steht,
$R^5$ für Wasserstoff, Alkyl, Alkoxyalkyl, Alkylthioalkyl, Fluoralkyl, Cyanalkyl, Alkenyl, Alkinyl oder Cycloalkyl steht,
oder
$R^4$ und $R^5$ gemeinsam mit dem Stickstoffatom, an dem sie stehen, für einen unsubstituierten oder substituierten Heterocyclus stehen, welcher in der Alkylenkette durch weitere Heteroatome substituiert sein kann,
$R^6$ für Wasserstoff, Methyl oder Chlor steht und

Q für Schwefel oder -CH = CH- steht,
dadurch gekennzeichnet, daß man
a) eine aromatische oder heteroaromatische Hydroxylverbindung der allgemeinen Formel (II)

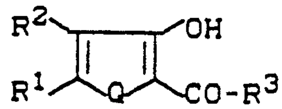
(II)

in welcher
$R^1$, $R^2$, $R^3$ und Q die oben angegebene Bedeutung haben,
oder deren Ammonium-, Alkali- oder Erdalkalisalze, mit einem Acylierungsmittel der Formel (III)

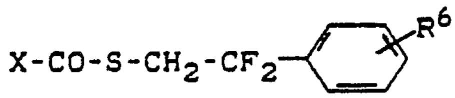
(III)

in welcher
$R^6$ die angegebene Bedeutung hat und
X für eine Abgangsgruppe steht,
umsetzt
oder daß man
b) Verbindungen der Formel (II) erst mit Phosgen in Gegenwart einer tertiären organischen Base umsetzt und dann den gebildeten Chlorcarbonyloxy-aromaten bzw. Chlorcarbonyloxy-heteroaromaten der Formel (IV)

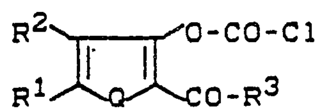
(IV)

in welcher
$R^1$, $R^2$, $R^3$ und Q die obengenannten Bedeutungen haben,
mit einem 2-Phenyl-2,2-difluorethylmercaptan der Formel (V)

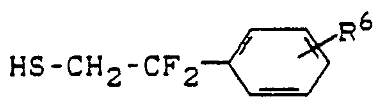
(V)

in welcher
$R^5$ die obengenannte Bedeutung hat,
in Gegenwart einer organischen oder anorganischen Base und in Gegenwart von Lösungs- und Verdünnungsmitteln umsetzt
oder daß man
c) für den Fall, daß in der Formel (I)
$R^2$ für Alkyl oder Phenyl steht,
Q für Schwefel steht,
$R^6$ für Wasserstoff, Methyl oder Chlor steht und
$R^1$ und $-COR^3$ Estergruppen mit ungleichen Resten sind,
ein 4-Hydroxythiophenderivat der Formel (IIa)

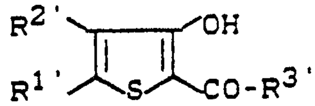
(IIa)

in welcher
$R^1$ für Alkoxycarbonyl, Alkoxyalkoxycarbonyl, Alkylthioalkoxycarbonyl, Halogenalkoxycarbonyl, Cyanalkoxycarbonyl, Alkenoxycarbonyl, Alkinoxycarbonyl, Cycloalkyloxycarbonyl oder Cycloalkylalkoxycarbonyl steht,
$R^{2\prime}$ für Alkyl oder Phenyl steht und
$R^{3\prime}$ für Alkoxy, Alkoxyalkoxy, Alkylthioalkoxy, Fluoralkoxy, Cyanalkoxy, Alkenoxy, Alkinoxy, Cycloalkyloxy oder Cycloalkylalkoxy steht,
mit der Maßgabe, daß $R^1$ und $COR^{3\prime}$ für gleiche Estergruppen stehen, mit wäßrig alkoholischer Alkalilauge im Molverhältnis 1:2 partiell zu 2-Carboxy-4-hydroxy-5-carbonsäureestern der Formel (IIb)

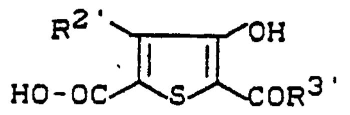

(IIb)

in welcher ,
$R^{2\prime}$ und $R^{3\prime}$ die bei der Formel (IIa) angegebenen Bedeutungen haben,
verseift und die Verbindungen der Formel (IIb) nach ihrer Isolierung mit einem 2-Phenyl-2,2-difluorethylthiolcarbonylhalogenid der Formel (III) in Gegenwart einer tertiären organischen Base zu Verbindungen der Formel (VI)

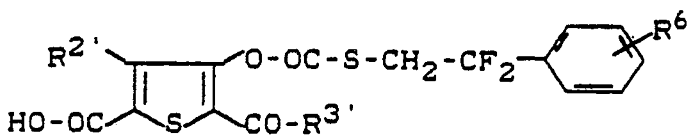

(VI)

in welcher
$R^{2\prime}$, $R^{3\prime}$ und $R^6$ die oben angegebenen Bedeutungen haben,
umsetzt und die so erhaltenen Verbindungen der Formel (VI) zu Säurechloriden der Formel (VII)

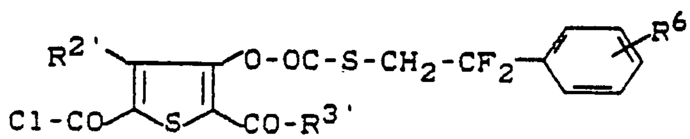

(VII)

in welcher
$R^{2\prime}$, $R^{3\prime}$ und $R^6$ die angegebenen Bedeutungen haben,
umsetzt und diese Verbindungen in letzter Stufe mit einem Alkohol der Formel (VIII)
$R^1$ OH (VIII)
in welcher
$R^1$ für Alkyl, Alkoxyalkyl, Alkylthioalkyl, Halogenalkyl, Cyanalkyl, Alkenyl, Alkinyl, Cycloalkyl oder Cycloalkylalkyl steht,
in Gegenwart einer organischen Base verestert.

7. Schädlingsbekämpfungsmittel, gekennzeichnet durch einen Gehalt an mindestens einem substituierten 2-Phenyl-2,2-difluorethylthiolcarbonat der Formel (I) nach den Ansprüchen 1 und 6.

8. Verwendung von substituierten 2-Phenyl-2,2-difluorethylthiolcarbonaten der Formel (I) nach den Ansprüchen 1 und 6 zur Bekämpfung von Schädlingen.

9. Verfahren zur Bekämpfung von Schädlingen, dadurch gekennzeichnet, daß man mindestens ein substituiertes 2-Phenyl-2,2-difluorethylthiolcarbonat der Formel (I) nach den Ansprüchen 1 und 6 auf Schädlinge und/oder ihren Lebensraum einwirken läßt.

10. Verfahren zur Herstellung von Schädlingsbekämpfungsmitteln, dadurch gekennzeichnet, daß man substituierte 2-Phenyl-2,2-difluorethylthiolcarbonate der Formel (I) nach den Ansprüchen 1 und 6 mit Streckmitteln und/oder oberflächenaktiven Mitteln vermischt.